# EUROPEAN PATENT APPLICATION

(11) **EP 0 541 830 A1**
(43) Date of publication of application: **19.05.1993**
(21) Application number: 91119103.9
(22) Date of filing: 09.11.1991
(51) Int. Cl.: A61K 7/00, A61K 7/48, A61K 47/00

(54) **Absorption base containing sucrose ester**

(71) Applicant: ISP Van Dyk Inc., Belleville, NJ 07109 (US)
(72) Inventor: Bergerhausen, Heinrich, W-5000 Köln 41 (DE)
(74) Representative: Fechner, Joachim, Dr.-Ing.

(57) **Abstract**

The absorption base for producing water-in-oil emulsions for cosmetic or pharmaceutical preparations comprises at least one fat alcohol ester as the oil component and at least one emulsifier from the group consisting of partial esters of polyvalent alcohols with fatty acids and is characterized in that it contains i. an emulsifier mixture of a) a di-, tri- or higher ester of sucrose with at least one fatty acid from the group consisting of the saturated fatty acids and oleic acid, or a mixture of such esters, and b) at least one oil-soluble fatty acid ester of sorbitan as co-emulsifier, ii. at least one consistency regulator from the group consisting of vegetable and animal waxes and consistency regulating fat alcohols, and iii. peanut oil as an additional oil component. The absorption base serves for preparing cosmetic or pharmaceutical water-in-oil emulsions the components of which are naturally regrowing products and/or are derived from such products. The absorption base is substantially hydrocarbon-free and also free from lanolin and lanolin derivatives.

## Description

The invention relates to an absorption base for the production of water-in-oil emulsions for cosmetic or pharmaceutical preparations.

Absorption bases are bases of ointments or creams which can absorb considerable quantities of water while forming water-in-oil emulsions, and serve for the production of cosmetic or pharmaceutical creams. Absorption bases are purchased as intermediates by producers of cosmetics and drugs and are processed by them with the addition of active agents and water to emulsions to form the final product. In view of the various final products and processing conditions the absorption bases have to fulfil high requirements with respect to the formation and stability of the emulsions to be prepared from them.

Absorption bases on the basis of petroleum jelly, paraffin, ceresin, mineral oil etc. are known to a large extent. Lanolin alcohols, lanolin derivatives, cholesterol and cholesterol esters primarily serve as emulsifiers for these oil components. Such bases having a hydrocarbon basis can be relatively easily emulsified. With the tendency to use natural products in cosmetics and with the interest to use increasing quantities of natural regrowing raw materials, there is a certain hesitation on the part of consumers to purchase skin care products based on hydrocarbons. Furthermore, as a result of the use of insecticides in sheep farming the lanolin products often contain small insecticide quantities which are difficult to remove and represent an allergy potential.

As far as paraffin-free and lanolin-free absorption bases for preparing cosmetic water-in-oil emulsions are known the stability of such emulsions is limited and often not sufficient. As examples pentaerythritol fatty acid ester citrate or glycerol sorbitol fatty acid ester are used as emulsifiers for these absorption bases.

From Cosmetics & Toiletries 95 (1981) p. 73-76 the use of emulsifier mixtures comprising sucrose esters for the formulation of cosmetic water-in-oil creams is known. The emulsifier mixture of the specified creams consists of sucrose stearates and sorbitan sesquioleate in addition to lanolin. The oil phase mainly consists of paraffin and mineral oil. Because of the hydrocarbon phase the emulsification goes without problems.

From the Japanese Patent Application 59-144715 an ointment base in the form of an oil-in-water emulsion is known which in addition to a triglyceride of a saturated mid-size chain fatty acid contains a sucrose fatty acid ester of an addition molar number of 4-8 and a sucrose fatty acid ester of an addition molar number of 1-3.

Finally from the Japanese Patent Application 60-76543 a cosmetic preparation in the form of an oily gel composition is known. The gel composition contains sucrose fatty acid esters and the fatty acid ester of a starch hydrolyzate. The fatty acids of the sucrose esters have at least 12 carbon atoms, and the monoester content of the sucrose esters is 10 weight percent at maximum.

It is an object of the present invention to provide an absorption base for producing water-in-oil emulsions of high stability for cosmetic or pharmaceutical preparations.

It is a further object of the invention to provide a hydrocarbon-free absorption base for producing stable water-in-oil emulsions which is also free from lanolin, lanolin alcohols and other lanolin derivatives.

Furthermore, it is an object of the invention to provide an absorption base for preparing cosmetic or pharmaceutical water-in-oil emulsions the components of which have a high content of naturally regrowing products and/or are derived from such products.

It is a further object of the invention to provide an absorption base which can be easily processed to cosmetic or pharmaceutical ointments and creams of complicated and various compositions and of sufficient emulsion stability so that the final processors have plenty of scope for their formulation.

Finally it is an object of the invention to provide an absorption base which is based on a vegetable oil and fatty ester oil, capable of forming long-term stable water-in-oil emulsions in spite of emulsification difficulties generally resulting from the polarity of said oils.

Starting from an absorption base comprising at least one fat alcohol ester as the oil component and at least one emulsifier from the group consisting of the partial esters of polyvalent alcohols with higher fatty acids, the absorption base of the invention is characterized in that it contains
i. an emulsifier mixture of
   a) a di-, tri- or polyester of sucrose with at least one fatty acid from the group consisting of the saturated fatty acids and oleic acid, or a mixture of such esters, and
   b) at least one oil-soluble fatty acid ester of sorbitan as a co-emulsifier,
ii. at least one consistency regulator from the group consisting of vegetable and animal waxes and consistency regulating fat alcohols, and
iii. peanut oil as an additional oil component.

Unlike the known absorption bases the stabilisation of the water-in-oil emulsion to be formed is achieved by the combined action of at least two emulsifiers one of which is a sucrose fatty acid ester, and at least one consistency regulator, and peanut oil as an additional oil component. The applicant has found that sucrose esters alone do not result in sufficiently long-term stable water-in-oil emulsions with peanut oil and the fat-like ester oils used in this invention. Likewise, a sufficiently long-term stability of water-in-oil emulsions cannot be achieved in the used oil phase with the fatty acid esters of sorbitan alone. This applies to oil phases which are substantially paraffin-free and contain polar oil components. Rather, our research has shown that a sufficient long-term stability of the formed emulsion can be achieved only by the combined action of the sucrose fatty acid esters together with the sorbitan fatty acid esters and the consistency regulating agents. In this connection the consistency regulators have the function to increase the rigidity of the interfacial films when the emulsion and its interfaces have been formed, to impart a firm consistency to the emulsion, and to reduce the external influences changing or impairing the emulsion structure.

Preferably, the emulsifier mixture comprises sucrose ester with at least one saturated fatty acid having an even number of carbon atoms from 12 to 28. Especially the emulsifier mixture comprises sucrose ester with at least one saturated fatty acid having an even number of carbon atoms from 16 to 22. Both components of these sucrose esters the sucrose and the fatty acids are derived from natural regrowing sources.

Owing to their production the sucrose esters used in accordance with the invention are generally present as a mixture of di-esters, tri-esters and higher esters and can be directly used as the mixture. The ester mixture can contain minor amounts of mono-esters. The sucrose ester mixture generally does not contain more than 10 weight percent mono-esters. Suitable fatty acids for the esterification of the sugar are palmitic acid, stearic acid, isostearic acid, arachidic acid, behenic acid and oleic acid. Fatty acids with 16 or 18 carbon atoms are the preferred acid components of the sucrose esters. Consequently, palmitic acid, stearic acid and oleic acid are the most preferred acids.

The sorbitan fatty acid esters used as the co-emulsifier are water-in-oil emulsifiers as are the used sucrose esters. They have HLB values in the range from 1 to 7, preferably from 1 to 5.

Suitable consistency regulating agents are beeswax, carnauba wax, shellac wax etc. as well as the consistency regulating fat alcohols. The preferred consistency regulating fat alcohols are those with 14 to 18 carbon atoms. Preferred examples of alcohols which can be used are myristyl alcohol, cetyl alcohol, stearyl alcohol, isostearyl alcohol or mixtures of these alcohols such as cetyl alcohol and stearyl alcohol (Lanette O).

According to a preferred embodiment of the absorption base the sucrose ester mixture comprises a mixture of palmitate and stearate in a weight ratio in the range from 10:90 to 50:50. The most preferred weight ratio is about 30:70. A mixture of sucrose fatty acid esters of this composition is available from tallow by transesterification after having hydrogenated the oleic acid portion. A mixture of the sucrose esters is generally used in view of the costs. Preferably the mixture of the sucrose esters is substantially free from sucrose mono-esters because the mono-esters are oil-in-water emulsifiers and yield no advantage or are at least irrelevant for the processing of the absorption base and the stability of the emulsion. The portion of the tri-esters and the higher esters in the sucrose ester mixture is generally at least 35 weight percent.

Preferably the co-emulsifier is a sorbitan ester of the general formula

C₆H₈O(OH)ₙ(OR)₄₋ₙ

in which n is an integer from 1 to 3, and R means the acid residue of a fatty acid having 16 or 18 carbon atoms, namely palmitoyl, stearoyl, isostearoyl or oleoyl. Particularly, R means isostearoyl or oleoyl. These emulsifiers are commercially available under different trade names as for instance Span and Arlacel. Conveniently, the emulsifier mixture comprises polyalkylene oleate laurate and/or polyglyceryl oleate as additional co-emulsifiers.

The preferred embodiment of the absorption base of the invention contains a mixture of beeswax, cetyl alcohol and stearyl alcohol as consistency regulators. Generally the beeswax forms the major part of the mixture. Thus, the mixture of the consistency regulators can comprise 50 to 80 weight percent beeswax and 50 to 20 weight percent of a mixture consisting of approximately equal parts of cetyl alcohol and stearyl alcohol.

According to the preferred embodiment of the absorption base the oil components comprise at least one fat alcohol ester of a fatty acid the fatty acid moiety of which has 8 to 36 carbon atoms and can be substituted by a stearoyl group, and the fat alcohol moiety of which has 6 to 20 carbon atoms. This oil component is used to give desirable properties such as a good spreading property, skin emollient property and sliding property to the water-in-oil emulsion to be formed.

In order to achieve a good spreading property of the emulsion to be formed fat alcohol esters of fatty acids are used the alcoholic moiety of which is derived from a chain-branched alcohol with 10 to 20 carbon atoms. Suitable alcohols are isodecanol and 2-ethylhexanol. Preferred esters improving the spreading property of the water-in-oil emulsion to be formed are isodecyl laurate and 2-ethylhexyl palmitate stearate.

With an especially preferred embodiment of the absorption base of the invention the oil components consist of a mixture of 2-octyldodecyl-12-stearoyl stearate, isodecyl laurate and peanut oil. 2-Octyldodecyl-12-stearoyl stearate is similar to paraffins owing to its long alkyl chains and its high number of carbon atoms. It imparts the property to the emulsions to form a long lasting, but not occluding film on the skin which generates a pleasant skin feeling without being greasy.

The absorption base according to the invention preferably consists of 10 to 30 weight percent sucrose esters, 10 to 20 weight percent co-emulsifier(s), 10 to 30 weight percent consistency regulating agent(s), and 20 to 70 weight percent peanut oil and fat alcohol ester(s). The relatively high content of emulsifiers and consistency regulators assures the processing of the absorption base to stable cosmetic or pharmaceutical final products with a greater variety of active additives and quantities of added water. The water number of the absorption bases of the invention which are free from lanolin and lanolin derivatives can amount up to 515 and consequently is substantially higher than that of lanolin. An especially preferred absorption base contains from 15 to 25 weight percent sucrose esters, from 10 to 16 weight percent co-emulsifier(s), from 15 to 20 weight percent consistency regulator(s) and from 39 to 60 weight percent peanut oil and fat alcohol ester(s). Generally, the used emulsifiers mainly consist of sucrose ester(s). The portion of the sucrose ester(s) in the emulsifier mixture can be 50 to 85 weight percent, preferable 50 to 70 weight percent.

A typical particularly preferred absorption base of the invention consists of 18 to 22 weight percent sucrose stearates and palmitates, 7 to 9 weight percent polyglyceryl-4-oleate and PEG-8-propylene glycol cocoate, 4 to 6 weight percent sorbitan isostearate, 10 to 14 weight percent beeswax, 5 to 7 weight percent cetyl alcohol and stearyl alcohol in equal parts, 20 to 26 weight percent 2-octyl dodecyl-12-stearoyl stearate, 18 to 22 weight percent isodecyl laurate and 5 to 7 weight percent peanut oil.

The absorption base of the invention is prepared by intensive mixing of the components. The absorption base is stable; neither separations nor chemical reactions between the components occur.

The further processing of the absorption base of the invention to water-in-oil emulsions or final cosmetic or pharmaceutical products is carried out in a known manner. The absorption base and the water phase to be emusified are heated to about 80-90 °C. Oil-soluble active agents or additives are added to the absorption base, water-soluble components are added to the water phase. The heated water phase is added to the heated absorption base with agitation. After cooling to about 40 °C the perfume oils and, if desired, other heat-sensitive substances are added. After cooling to room temperature the emulsion is finally homogenised by rolling, grinding or pressing through nozzles. The emulsion stability of the obtained product is at least 3 years.

The absorption base of the invention is a hydrocarbon-free base with high content of naturally regrowing components and/or of derivatives of naturally regrowing raw materials. Consequently, the absorption base is a starting material for producing nature-cosmetic creams and ointments. The term "hydrocarbon-free" or "paraffin-free" used here-above is to indicate that no hydrocarbons themselves are used as components, particularly as oil components when forming the absorption base. As in some cases the used natural components contain small portions of hydrocarbons, e.g. beeswax may contain about 10 to 13 weight percent long-chain n-paraffins, these minor hydrocarbon quantities of biological origin may be present in the absorption base.

Due to the components in the base, especially to the high portion of sucrose esters, the cream film applied onto the skin preserves the moisture in the skin without the need of a moisture barrier layer as with the occlusion by a hydrocarbon film. A further advantage of the creams and ointments prepared from the absorption bases of the invention resides in the fact that the permeation of active agents through the skin is enhanced which results from the moisture preservation in the skin. If, however, in some cases an occlusion effect of the final cosmetic or dermatological preparation is desired it can be achieved by the addition of a paraffinic component when formulating the final product. As a further advantage the creams from the absorption base of the invention impart a pleasant skin feeling which results from the moisture preservation and skin emolliency on the one hand and from the protection of the lipid layer of the skin without an external excessive greasing on the other hand.

### Example

An absorption base was prepared by mixing the following components:
- 20 weight percent: sucrose ester mixture consisting of stearate and palmitate,
- 5 weight percent: sorbitan monoisostearate (Eramuls 60),
- 8 weight percent: polyglyceryl-4-oleate and PEG-8-propylene glycol cocoate,
- 6 weight percent: cetyl alcohol and stearyl alcohol in equal parts (Lanette O),
- 12 weight percent: beeswax,
- 20 weight percent: isodecyl laurate,
- 23 weight percent: 2-octyldodecyl-12-stearoyl stearate (Ceraphyl 847),
- 6 weight percent: peanut oil.
The sucrose ester mixture consisted of approx. 70% stearate and approx. 30% palmitate. It was substantially free from mono-esters, and its HLB value was about 1.

In order to prepare an all-purpose w/o cream from the obtained absorption base, primarily the oil phase is formed from the following components:
- 35 parts by weight: of the absorption base,
- 2 parts by weight: ester of polyethylene glycol with saturated fatty acid, HLB 6.4 (Arlacel 989),
- 6.3 parts by weight: 2-ethyl hexyl palmitate (Ceraphyl 368),
- 7.5 parts by weight: decyl oleate (Ceraphyl 140).
The mixture of these components was stirred at 80 °C until its homogeneity was achieved (phase A). A quantity of deionized water needed for filling up to 100 parts by weight was heated together with 1.0 part by weight calcium salt of D-pantothenic acid to 85 °C (phase B) and added to the phase A and stirred until homogeneity was reached. Then the homogeneous mixture phase A+B was cooled down in a water bath to 35 to 40 °C with stirring. Then small quantities of preservation agents and perfume were added, and the obtained emulsion was again homogenized.

The stability of the obtained emulsion was determined by the following tests:
- CT1:: The emulsion was centrifuged for 30 minutes with 4000 rpm and visually checked on phase separation after 24 hours.
- CT7:: The emulsion was centrifuged for 30 minutes with 4000 rpm and visually checked on phase separation after 7 days.
- RT:: The emulsion was left standing for more than 2 months at room temperature and visually checked on phase separation.
- HC:: The emulsion was stored in a heating chamber at 38°C for more than 2 months and then visually checked on phase separation.
The results of these stability tests are quoted in the table here-below.

### Comparative Example 1

An absorption base of the same composition as in the preceding example was prepared with the exception that corn oil was used instead of peanut oil. The emulsion was prepared in the same way as specified in the example. The emulsion was subjected to the same stability tests as the emulsion of the preceding example. The test results are quoted in the table.

### Comparative Example 2

An absorption base of the composition quoted in the example was prepared with use of a higher molecular ester mixture which mainly consisted of pentaerythritol fatty acid ester and citric acid ester (Dehymuls E), instead of sorbitan monoisostearate as co-emulsifier. The emulsion prepared as for the rest in the same way and with the same components was subjected to the same stability tests. The results are quoted in the table.

### Comparative Example 3

An absorption base of the same composition as that of the example was prepared with the exception that the co-emulsifier sorbitan monoisostearate and the consistency agent cetyl alcohol/stearyl alcohol (Lanette O) were omitted. As for the rest the procedure was the same as in the example. The results of the stability tests are quoted in the table.

### Comparative Example 4

An absorption base of the same composition as in the example was prepared with the exception that the co-emulsifier sorbitan monoisostearate was omitted and the peanut oil was replaced by the same quantity of paraffin oil. The emulsion formed in the same way as in the example was subjected to the same stability tests. The results are given in the table.

| Stability tests | CT1 | CT7 | RT | HC |
|---|---|---|---|---|
| Example | o | o | o | o |
| Comp. Example 1 | o | + | o | o |
| Comp. Example 2 | o | + | o | o |
| Comp. Example 3 | o | + | + | ++ |
| Comp. Example 4 | + | ++ | + | ++ |
| o : stable emulsion + : minor oil separation ++ : oil separation | | | | |

## Claims

1. Absorption base for producing water-in-oil emulsions for cosmetic or pharmaceutical preparations, comprising at least one fat alcohol ester as the oil component and at least one emulsifier from the group consisting of partial esters of polyvalent alcohols with fatty acids, characterized in that the absorption base contains
i. an emulsifier mixture of
a) a di-, tri- or higher ester of sucrose with at least one fatty acid from the group consisting of the saturated fatty acids and oleic acid, or a mixture of such esters, and
b) at least one oil-soluble fatty acid ester of sorbitan as co-emulsifier,
ii. at least one consistency regulator from the group consisting of vegetable and animal waxes and consistency regulating fat alcohols, and
iii. peanut oil as an additional oil component.

2. Absorption base according to claim 1, characterized in that the emulsifier mixture comprises sucrose ester with at least one saturated fatty acid having an even number of carbon atoms from 12 to 28.

3. Absorption base according to claim 1 or 2, characterized in that the emulsifier mixture comprises sucrose ester with at least one saturated fatty acid having an even number of carbon atoms from 16 to 22.

4. Absorption base according to any of the claims 1 to 3, characterized in that the sucrose ester mixture comprises a mixture of palmitate and stearate in a weight ratio in the range from 10:90 to 50:50.

5. Absorption base according to any of the claims 1 to 4, characterized in that the emulsifier mixture is substantially free from sucrose mono-esters.

6. Absorption base according to any of the claims 1 to 5, characterized in that the co-emulsifier is a sorbitan ester of the general formula
C₆H₈O(OH)ₙ(OR)₄₋ₙ
in which n is an integer from 1 to 3 and R means palmitoyl, stearoyl, isostearoyl or oleoyl.

7. Absorption base according to any of the claims 1 to 6, characterized in that the emulsifier mixture comprises polyoxyalkylene oleate laurate as an additional co-emulsifier.

8. Absorption base according to any of the claims 1 to 7, characterized in that the consistency regulating fat alcohol is selected from fat alcohols with 14 to 18 carbon atoms.

9. Absorption base according to any of the claims 1 to 8, characterized in that it contains a mixture of beeswax, cetyl alcohol and stearyl alcohol as consistency regulators.

10. Absorption base according to any of the claims 1 to 9, characterized in that the oil component comprises at least one fat alcohol fatty acid ester, the fatty acid moiety of which has 8 to 36 carbon atoms and can bear a stearoyl group as a substituent, and the fat alcohol moiety of which has 6 to 20 carbon atoms.

11. Absorption base according to claim 10, characterized in that the fat alcohol moiety is derived from a chain-branched alcohol with 10 to 20 carbon atoms.

12. Absorption base according to any of the claims 1 to 11 characterized in that it contains a mixture of 2-octyldodecyl-12-stearoyl stearate and isodecyl laurate as fat alcohol esters.

13. Absorption base according to any of the claims 1 to 12, characterized by 10 to 30 weight percent sucrose ester(s), 10 to 20 weight percent co-emulsifier(s), 10 to 30 weight percent consistency regulator(s) and 20 to 70 weight percent peanut oil and fat alcohol ester(s) as the oil component.

14. Absorption base according to claim 13, characterized by 15 to 25 weight percent sucrose ester(s), 10 to 16 weight percent co-emulsifier(s), 15 to 20 weight percent consistency regulator(s) and 39 to 60 weight percent peanut oil and fat alcohol ester(s) as the oil components.

15. Absorption base according to any of the claims 1 to 14, characterized by 18 to 22 weight percent sucrose stearate and palmitate, 7 to 9 weight percent polyglyceryl-4-oleate and PEG-8-propylene glycol cocoate, 4 to 6 weight percent sorbitan isostearate, 10 to 14 weight percent beeswax, 5 to 7 weight percent cetyl alcohol and stearyl alcohol, 20 to 26 weight percent 2-octyldodecyl-12-stearoyl stearate, 18 to 22 weight percent isodecyl laurate and 5 to 7 weight percent peanut oil.

16. Absorption base according to any of the claims 1 to 15, characterized in that it is substantially free from hydrocarbons.
